# EUROPEAN PATENT APPLICATION

(11) **EP 4 721 644 A1**
(43) Date of publication of application: **08.04.2026**
(21) Application number: 24818980.5
(22) Date of filing: 05.03.2024
(51) Int. Cl.: A61B 3/135

(54) **OPHTHALMIC DEVICE**

(30) Priority: 05.06.2023 JP 2023092438
(71) Applicant: Topcon Corporation, Tokyo 174-8580 (JP)
(72) Inventor: YAMAMOTO Satoshi, Tokyo 174-8580 (JP)
(74) Representative: Louis Pöhlau Lohrentz
(86) International application number: PCT/JP2024/008254
(87) International publication number: WO 2024/252741

(57) **Abstract**

Provided is an ophthalmic device that makes it possible to easily and selectively attach a specific light irradiation system for emitting specific light. A slit-lamp microscope (100), which is an example of this ophthalmic device, comprises: a body part (10) having an illumination system (30) for irradiating an eye (E) being examined of a subject with illumination light and an observation system (40) for observing return light from the eye (E) being examined; and an imaging unit (60) that is detachably attached to the body part (10) . The imaging unit (60) has an imaging section (61) for acquiring an image of the eye (E) being examined and a background illumination system (80) for irradiating the eye (E) being examined with background light different from the illumination light.

## Description

### Technical Field

The present disclosure relates to an ophthalmic device.

### Background Art

An ophthalmic device, commonly referred to as slit-lamp microscope or simply as a "slit lamp," is conventionally known. Such a device typically comprises a slit lamp that emits slit light; a mirror unit having a reflecting mirror or a prism that directs slit light emitted from the slit lamp toward an eye under examination; a microscope unit for observing the eye; and a background illumination system (also referred to as specific light irradiation system) that irradiates the eye with background light (specific light) to illuminate the background of the slit light (see, for example, Patent Literature 1) . Some ophthalmic devices may further include an imaging device that captures an image of an eye being examined which is illuminated with the specific light.

### Citation List

### Patent Literature

Patent Literature 1: JP 5747924 B

### Summary

### Technical Problem

Some users of the ophthalmic device observe the eye without using the specific light, and therefore such devices do not require a specific light irradiation system. In conventional ophthalmic devices, however, the specific light irradiation system is typically integrated with the microscope unit or the mirror unit, and the operation unit for operating the specific light irradiation system is also integrated with the pedestal portion of the ophthalmic device. Such a configuration prevents flexible attachment and detachment of the specific light irradiation system in accordance with user needs.

The present disclosure has been made in view of the above problems and aims to provide an ophthalmic device that allows easy and selective attachment of the specific light irradiation system for emitting specific light and an operation unit thereof.

### Means for Solving the Problems

To achieve the aforementioned objective, an ophthalmic device comprising a main unit including an illumination system configured to emit illumination light toward an eye of a subject under examination, and an observation system configured to allow an examiner to observe return light from the eye; and an imaging unit configured to be detachably attached to the main unit. The imaging unit comprises an imaging module configured to acquire an image of the eye; and a specific light irradiation system configured to emit specific light, different from the illumination light, toward the eye.

### Advantageous Effect of Invention

The ophthalmic device configured as described above enables the imaging unit to be attached to and detached from the main unit, thereby facilitating easy and selective attachment of the specific light irradiation system that emits specific light. Accordingly, users can selectively use the ophthalmic device with or without the specific light irradiation system.

### Brief Description of the Drawings

[FIG.1] FIG. 1 is a right-side view showing an overall configuration of the ophthalmic device according to Embodiment 1 and a perspective view of a movement mechanism.
[FIG.2A] FIG. 2A is an explanatory diagram illustrating a wiring condition and an imaging unit detachable from the ophthalmic device according to Embodiment 1, and is a left-side view schematically showing an ophthalmic device to which an imaging unit is not attached.
[FIG.2B] FIG. 2B is an explanatory diagram illustrating a wiring condition and an imaging unit detachable from the ophthalmic device according to Embodiment 1, and is a left-side view schematically showing an ophthalmic device to which an imaging unit is attached.
[FIG.3] FIG. 3 is a rear view showing the vicinity of a background light switching unit of the ophthalmic device according to Embodiment 1 viewed from the examiner side, and is an explanatory drawing illustrating a state of the background light switching unit during switching of background light.
[FIG.4] FIG. 4 is a front view showing a background illumination system and the vicinity of objective lens of the ophthalmic device according to Embodiment 1 viewed from the examiner side.
[FIG.5] FIG. 5 is a perspective view showing an overall configuration of an ophthalmic device according to Embodiment 2.
[FIG.6] FIG. 6 is an explanatory diagram illustrating a wiring condition and an imaging unit detachable from the ophthalmic device according to Embodiment 2, and is a left-side view schematically showing an ophthalmic device to which an imaging unit is attached. Description of Embodiments

### (Embodiment 1)

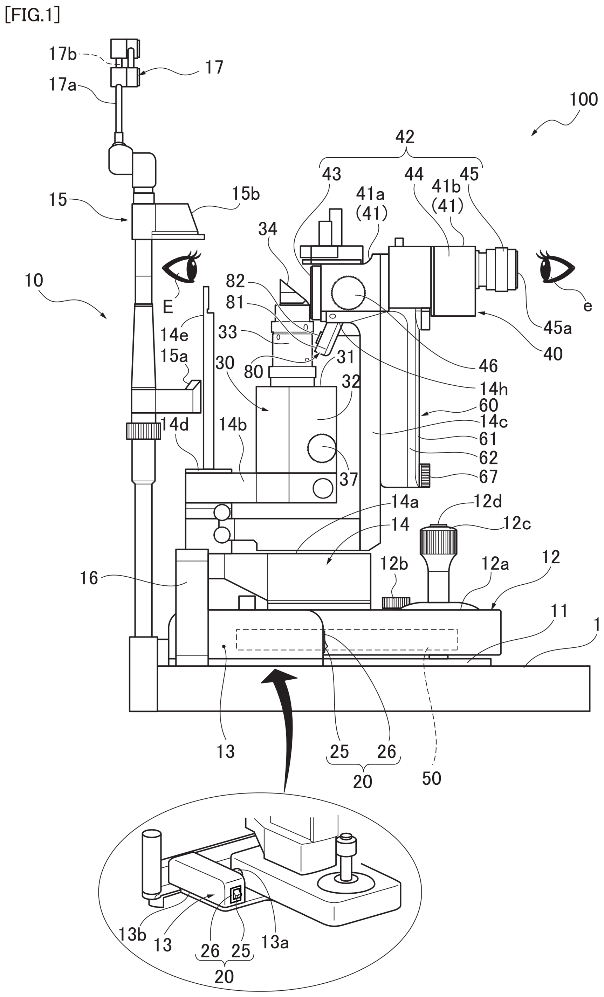

A slit-lamp microscope 100 according to Embodiment 1 of the ophthalmic device of the present disclosure is described below with reference to FIGS. 1 to 4. The slit-lamp microscope 100 shown in FIG. 1 is an ophthalmic device that uses slit-shaped illumination light, also referred to as "slit light", to acquire a cross-sectional image of a cornea by optically sectioning the cornea of an eye E.

The slit-lamp microscope 100 according to Embodiment 1 mainly comprises a main unit 10, a power supply unit 20, and an imaging unit 60, as shown in FIG. 1. The main unit 10 mainly comprises an illumination system 30, an observation system 40, and a control device 50. The imaging unit 60 mainly comprises an imaging module 61 and a background illumination system 80, which serves as an example of the specific light irradiation system.

The slit-lamp microscope 100 according to Embodiment 1 is a Zeiss-type (also referred to as a Littman-type) slit-lamp microscope in which the illumination system 30 is disposed below the observation system 40, thereby providing a more compact configuration than that of a slit-lamp microscope 100A according to Embodiment 2 described later.

The main unit 10 is mounted on an optical table 1. As mentioned above, the main unit 10 comprises the illumination system 30, the observation system 40, and the control device 50. The main unit 10 further comprises a base 11 mounted on the upper side of the optical table 1, a pedestal 12 provided on the upper side of the base 11, a movement mechanism 13 configured to move the pedestal 12 in the left-right direction, a supporting part 14 mounted on the upper side of the pedestal 12, a face support 15 supported on the base 11, and a pair of grips 16 provided on both left and right sides of the base 11.

The pedestal 12 comprises a pedestal 12a, a light control knob (light control operating unit) 12b, an operation lever (movement operating unit) 12c, and a capture button 12d provided on the upper side of the operation lever 12c. The pedestal 12 is configured to be moved relative to the base 11 by the movement mechanism 13 in the front-back direction and the left-right direction as viewed from an eye under examination. The movement mechanism 13 comprises a sliding bar 13a inserted into the pedestal 12a and a pair of fixed parts 13b that support the sliding bar 13a at both ends to allow the sliding bar 13a to move in the front-back direction. In this specification, the "front" direction of the front-back directions refers to a direction toward a subject (a patient under examination), and the "back" direction refers to a direction away from the subject and toward an examiner (a user of the device to examine the subject) ; The "left" direction of the left-right directions refers to the left-hand direction of the subject, and the right direction refers to the right-hand direction of the subject.

The operation lever 12c is configured to input operational instructions, such as instructions to move the pedestal 12. The capture button 12d is configured to input capture instructions to the imaging module 61 to capture an image. The operation lever 12c and the capture button 12d are each electrically connected to the control device 50. Using the operation lever 12c allows the examiner using the slit-lamp microscope 100 to control the device as follows: By gripping and fully moving the operation lever 12c to its end, the entire pedestal 12 is moved in the front-back and left-right directions by the movement mechanism 13. By performing a tilt operation, in which the examiner moves the operation lever 12c to an intermediate position and holds it there, the control device 50 controls the observation system 40 according to the direction and amount of the tilt operation to adjust the focus of the observation system 40. By rotating the operation lever 12c around its axis, the supporting part 14 provided on the upper side of the base 11 is raised and lowers in the vertical (up-down) direction according to the rotational direction and amount of the rotating operation by a lifting-and-lowering mechanism (not shown) . Using the capture button 12d, the control device 50 controls the imaging module 61 to capture an image of the eye E.

The light control knob 12b is configured to turn on and off the slit light of the illumination system 30 and to adjust its brightness (amount of light). By rotating the light control knob 12b, the illumination system 30 is turned on and off under control of the control device 50, and when turned on, the brightness of the illumination light is continuously adjusted according to the rotational direction and amount from the turned-off position.

The supporting part 14 comprises a stand 14a, a first supporting arm 14b, and a second supporting arm 14c. The stand 14a is provided on the upper side of the pedestal 12a. The first supporting arm 14b and the second supporting arm 14c are provided upright on the stand 14a and are configured to independently pivot about a common vertical axis 14d.

The supporting part 14 comprises a test rod 14e that is detachably received in a rotation-axis hole (not shown) provided on the vertical axis 14d. The test rod 14e serves to check the state of slit light emitted from the illumination system 30 and is removed when the examiner observes the eye E with the slit light.

The first supporting arm 14b supports the illumination system 30 at its upper end and is attached to an illumination-system housing 31 that houses optical components of the illumination system 30. The first supporting arm 14b is configured to be pivoted manually. Pivoting the first supporting arm 14b allows the examiner to revolve the illumination-system housing 31 around the eye E. Accordingly, the slit light can be directed toward the eye E in various directions. It should be noted that the first supporting arm 14b may be further configured to pivot in the vertical direction so that the slit light can be adjusted at elevational or depressional (upward and downward) angles relative to the eye E.

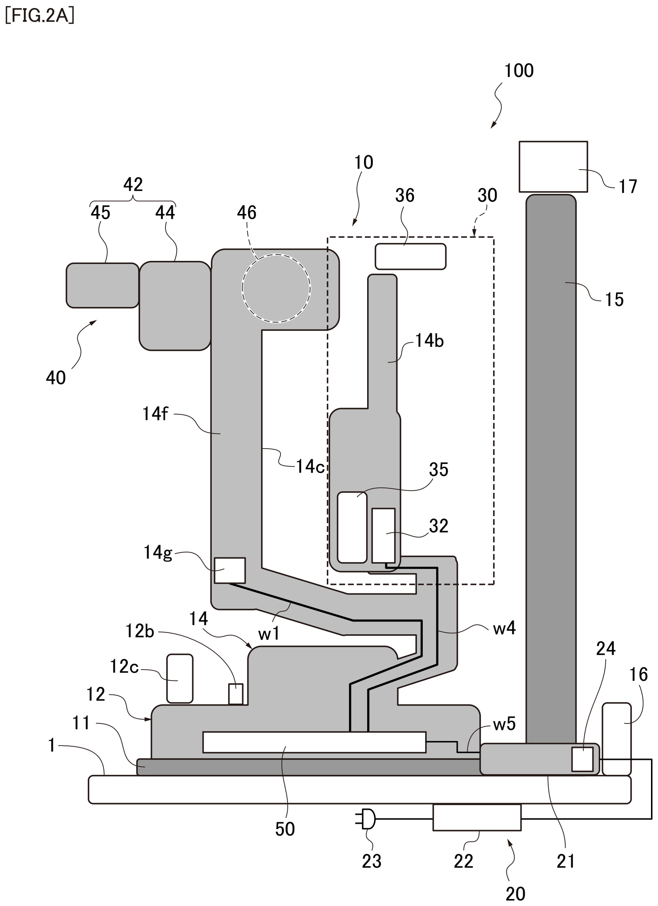

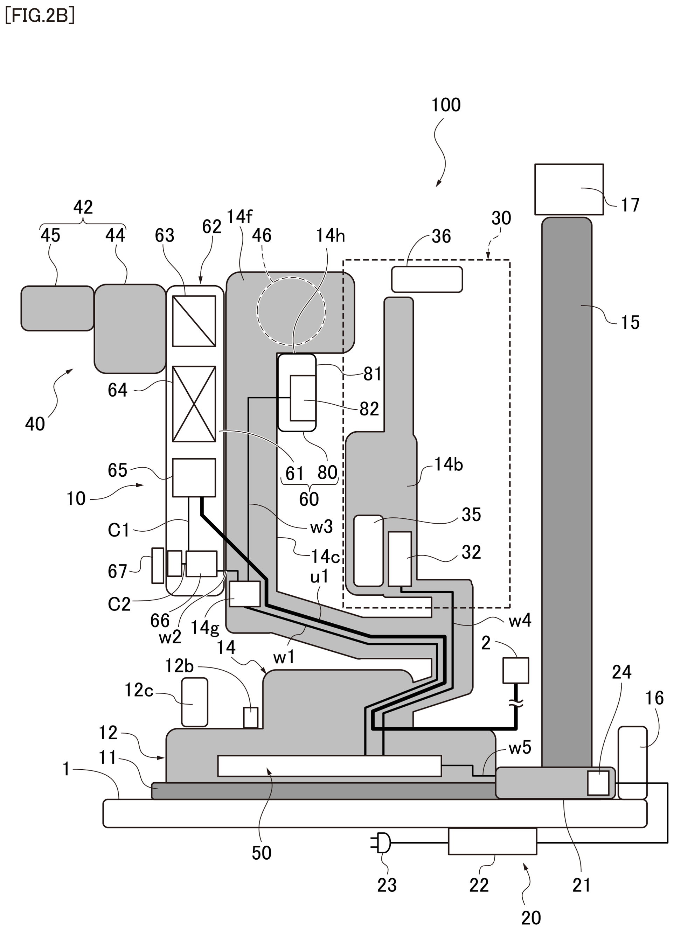

The second supporting arm 14c is formed as a tubular body having an L-shaped profile in side view and defines an accommodating space 14f inside that houses cables and wires (see FIGS. 2A and 2B). The second supporting arm 14c supports the observation system 40 at its upper end and is attached to an observation-system housing 41 that houses optical components of the observation system 40. The second supporting arm 14c supports the imaging unit 60, which is configured to be detachably attached below the observation system 40. The second supporting arm 14c is configured to be pivoted manually. Pivoting the second supporting arm 14c allows the examiner to revolve the observation system 40 and the imaging unit 60 around the first supporting arm 14b. Accordingly, the observation system 40 and the imaging unit 60 can be directed toward the eye E in various directions.

The second supporting arm 14c houses a relay board 14g in a lower part of the accommodating space 14f. The relay board 14g is electrically connected to a control device 50 housed in the pedestal 12a via a first power cable w1 extending through the accommodating space 14f and the pedestal 12a. The relay board 14g is also detachably connected to a second power cable w2 extending from the imaging module 61 of the imaging unit 60 (described below) and to a third power cable w3 extending from the background illumination system 80. The power cables w2 and w3 may also be housed in the accommodating space 14f.

The second supporting arm 14c is provided, on the subject-facing side, below the objective lens 43 of the observation system 40, with an attaching portion 14h to which the background illumination system 80 is attached. The attaching portion 14h has an aperture (not shown) to which one end of the background illumination system 80 is inserted, and the aperture is covered with a lid (not shown) when the background illumination system 80 is not in use. Alternatively, the attaching portion 14h may be configured to be composed of an input/output terminal, to which the background illumination system 80 is connected.

Alternatively, the first supporting arm 14b and the second supporting arm 14c may be configured to pivot automatically under electrical drive. In this case, the supporting part 14 may further comprise an actuator for generating a driving force to pivot the first supporting arm 14b and the second supporting arm 14c, and a transmission mechanism for transmitting the driving force. For example, the actuator may be embodied as a stepping motor (pulse motor), and the transmission mechanism may be embodied as a gear train or a rack-and-pinion mechanism.

The face support 15 is disposed in front of and facing the observation system 40. The face support 15 comprises a chin rest 15a on which the subject's chin is placed, and a forehead support 15b against which the subject's forehead is placed. In Embodiment 1, the examiner operates the slit-lamp microscope 100 to observe the eye E when the subject, facing the optical table 1, places the subject's face on the chin rest 15a and the forehead support 15b.

A fixation part 17, used for external fixation, is detachably mounted on the face support 15. The fixation part 17 comprises a fixation-lamp holding part 17a consisting of a flexible arm, and an external fixation lamp 17b (e.g., an LED light source) provided at the edge of the fixation-lamp holding part 17a to emit fixation light. Using the fixation-lamp holding part 17a allows the fixation part 17 to adjust the position of the external fixation lamp 17b and the irradiation direction of the fixation light as desired. Using the external fixation lamp 17b allows the fixation part 17 to guide the line of sight of the eye E, thereby enabling adjustment of the viewing direction of the eye E.

The pair of grips 16 are provided for the subject to hold with both hands during an examination. By holding the pair of grips 16, the subject can stabilize the body and maintain a stable posture, thereby facilitating proper examination.

The power supply unit 20 is configured to supply electric power to the slit-lamp microscope 100. The power supply unit 20 is connected to the control device 50 via a fifth power cable w5. The power supply unit 20 mainly comprises a switch box 21, an AC/DC converter 22, and an attachment plug 23. The switch box 21 comprises a switch board 24, a power switch 25, and a pilot lamp 26. The switch board 24 is a control device that controls turning on and off of the power of the slit-lamp microscope 100 in response to operations of the power switch 25. The power switch 25 turns the power supply on and off. The pilot lamp 26, which may be implemented as an LED light source, for example, is configured to indicate the status of the power supply of the slit-lamp microscope 100. The pilot lamp 26 turns on when the slit-lamp microscope 100 is in an electrified state, that is, in an activated state (the power is on), and turns off when the slit-lamp microscope 100 is in a non-electrified state, that is, in a stopped state (the power is off) .

The AC/DC converter 22 converts an AC voltage into a DC voltage. The attachment plug 23 is inserted into a power outlet (not shown) . When the examiner turns on the power using the power switch 25, the power supply unit 20 starts supplying electric power via the AC/DC converter 22 to the control device 50, and further to the illumination system 30, the observation system 40, and the imaging unit 60, all of which are connected to the control device 50.

The illumination system 30 emits slit light toward the eye E. The illumination system 30 mainly comprises a slit lamp 32 (e.g., an LED light source) housed in the illumination-system housing 31, and a deflection optical system 33. The illumination system 30 may comprise an exciter filter 35, a diffuser plate 36 (see FIG. 2B), or the like, which are selectively arranged on the optical path of the illumination system. The exciter filter 35 allows fluorescent observation of the cornea of the eye E when used together with a barrier filter, which will be described below. The diffuser plate 36 allows wider-area observation of the eye E by diffusing the illumination light emitted from the slit lamp 32.

The slit lamp 32 emits slit light toward the deflection optical system 33. The deflection optical system 33 is provided above the slit lamp 32. The deflection optical system 33 comprises a deflection optical element 34. The deflection optical element 34 is composed of a prism in Embodiment 1. The deflection optical element 34 deflects the slit light emitted from the slit lamp 32 toward the eye E, thereby illuminating the slit light toward the eye E. It should be noted that the deflection optical element 34 is not limited to a prism, and may be composed of a mirror (reflecting mirror) or the like to deflect the slit light by reflecting it toward the eye E. Also, the slit lamp 32 and the deflection optical system 33 are not limited to the configuration shown in FIG. 1, and may vary in shape, structure, and arrangement as long as they are applicable to a Zeiss-type slit-lamp microscope.

The slit lamp 32 is electrically connected to the control device 50 via a fourth power cable w4 housed in the first supporting arm 14b and the supporting part 14. With this configuration, the slit lamp 32 is supplied with electric power from the power supply unit 20 via the control device 50 and is controlled by the control device 50.

The brightness of the slit light can be adjusted and changed by operating the light control knob 12b provided on the pedestal 12a as described above. The width of the slit light can be adjusted and changed by operating a slit opening and closing knob (slit opening and closing operation unit) 37 provided with the illumination system 30. The slit light is band-shaped illumination light in which an illumination area is formed by partially blocking an irradiation area of a light source, and is used to illuminate the cornea and fundus of an eye under examination for observation.

The observation system 40 is used to observe return light (also referred to as "reflected light") from an eye under examination and is a microscope. It should be noted that "return light" includes not only slit light and background light reflected from an eye under examination, but also various types of light such as scattered light from the eye and its surroundings. In Embodiment 1, "return light" refers to these various types of light. The observation system 40 is attached to the upper part of the second supporting arm 14c as described above. The observation optical system 42, which guides return light from the eye E, is housed in the observation-system housing 41. The observation-system housing 41 according to Embodiment 1 consists of a first observation-system housing 41a disposed on the subject-facing side (front side) of the second supporting arm 14c and a second observation-system housing 41b disposed on the examiner-facing side (back side) of the second supporting arm 14c.

The observation optical system 42 comprises an objective lens 43, a pair of left and right optical relay 44, and a pair of left and right eyepieces 45. The objective lens 43 is a lens facing the eye E. The pair of left and right eyepieces 45 each comprises an ocular lens 45a that faces the examiner's corresponding left and right eyes e. The pair of left and right optical relays 44 each comprises optical components, such as a variable magnification optical system, an aperture, and a prism unit (not shown). Some of the objective lens 43 and the optical relay 44 (for example, a variable magnification optical system, an aperture, and the like) are housed in the first observation-system housing 41a, and the rest of the optical relay 44 (for example, a prism unit or the like) and the eyepieces 45 are housed in the second observation-system housing 41b. In some embodiments, the optical relay 44 may be configured such that all components are housed in the first observation-system housing 41a or in the second observation-system housing 41b.

The examiner can observe the eye E by looking into the pair of the eyepieces 45. The observation-system housing 41 is also provided on the left and right sides with an observation-magnification adjustment handle (observation-magnification adjustment operation unit) 46 for modifying the observation magnification.

The imaging unit 60 mainly comprises an imaging module 61 and the background illumination system 80 as described above, and is detachably attached to the second supporting arm 14c of the main unit 10. The imaging module 61 is attached to the examiner-facing side (back side) of the second supporting arm 14c. The background illumination system 80 is attached to the attaching portion 14h, which is provided on the subject-facing side (front side) of the second supporting arm 14c, below the objective lens 43 of the observation system 40.

The imaging module 61 receives return light from the eye E and captures an image of the eye E. The imaging module 61, as shown in FIG. 2B, mainly comprises an imaging module housing 62, an imaging module optical deflector 63, an imaging optical system 64, an imaging element 65, an LED board 66 serving as a control board, and a background light switching unit 67 serving as an operation unit. The imaging module housing 62 is attached to the second supporting arm 14c and is configured to be held between the second supporting arm 14c and the second observation-system housing 41b. The imaging module optical deflector 63, the imaging optical system 64, the imaging element 65, and the LED board 66 are housed in the imaging module housing 62.

With the imaging module 61 attached to the second supporting arm 14c, the imaging module optical deflector 63 is disposed within the optical path of the observation system 40, specifically in the optical path of either one of the pair of the optical relays 44, to deflect a portion of return light from the eye E out of the optical path of the observation system 40. The imaging module optical deflector 63, which is composed of a beam splitter according to Embodiment 1, reflects a portion of the return light to deflect the return light toward the imaging element 65 while transmitting the remainder of the return light. The imaging optical system 64 is composed of an optical element, such as an imaging lens, and collects the return light deflected by the imaging module optical deflector 63 to form an image on the imaging element 65.

The imaging element 65 is a photoelectric transducer that detects the return light collected by the imaging optical system 64, converts the detected light into an electric signal (image signal), and outputs the signal. The imaging element 65 is implemented using, for example, a charge-coupled device (CCD) image sensor or a complementary metal-oxide semiconductor (CMOS) image sensor.

In Embodiment 1, the imaging element 65 is connected to a personal computer (PC) 2 (see FIG. 2B) as an external device via a communication cable u1, to output an image signal to the PC 2. Accordingly, the PC 2 can store images captured by the imaging module 61. It should be noted that the control device 50 may receive the image signal and convert it into an image file or other medium representing the image, to store it in a storage device thereof.

The communication cable u1 is implemented, for example, as a USB cable, and is housed in the imaging module housing 62, the accommodating space 14f of the second supporting arm 14c, and the pedestal 12a. The communication cable u1, connected to the imaging element 65 at one end, extends from the pedestal 12a to the outside and is connected to the PC 2 at the other end, or is connected to an input/output terminal provided on the main unit 10, from which another USB cable (not shown) connects to the PC 2.

The LED board 66 is a circuit board provided with an LED light source. The LED board 66 is connected to the relay board 14g via the second power cable w2. The relay board 14g is connected to the control device 50 via the first power cable w1 as described above, and the control device 50 is connected to the power supply unit 20 via the fifth power cable w5. With this configuration, the LED board 66 is supplied with electric power from the power supply unit 20 via the relay board 14g and the control device 50, and is also controlled by the control device 50.

The LED board 66 is connected to the imaging element 65 via the first circuit cable C1, supplies electric power to the imaging element 65, and controls the imaging element 65 under the control of the control device 50. Here, pressing the capture button 12d triggers the control device 50 to control the LED board 66 via the relay board 14g, and the LED board 66 in turn controls the imaging element 65 to capture an image of the eye E.

The LED board 66 is also connected to a background light switching unit 67 via the second circuit cable C2. The LED board 66 is further connected to the background illumination system 80 via the second power cable w2, the relay board 14g, and the third power cable w3 housed in the accommodating space 14f. With this configuration, when the examiner operates the background light switching unit 67, the unit 67 outputs an electric signal based on the operation to the control device 50 via the LED board 66 and the relay board 14g. According to the electric signal, the control device 50 controls the background illumination system 80, including turning background light on and off, switching the background light, and adjusting its brightness (amount of light).

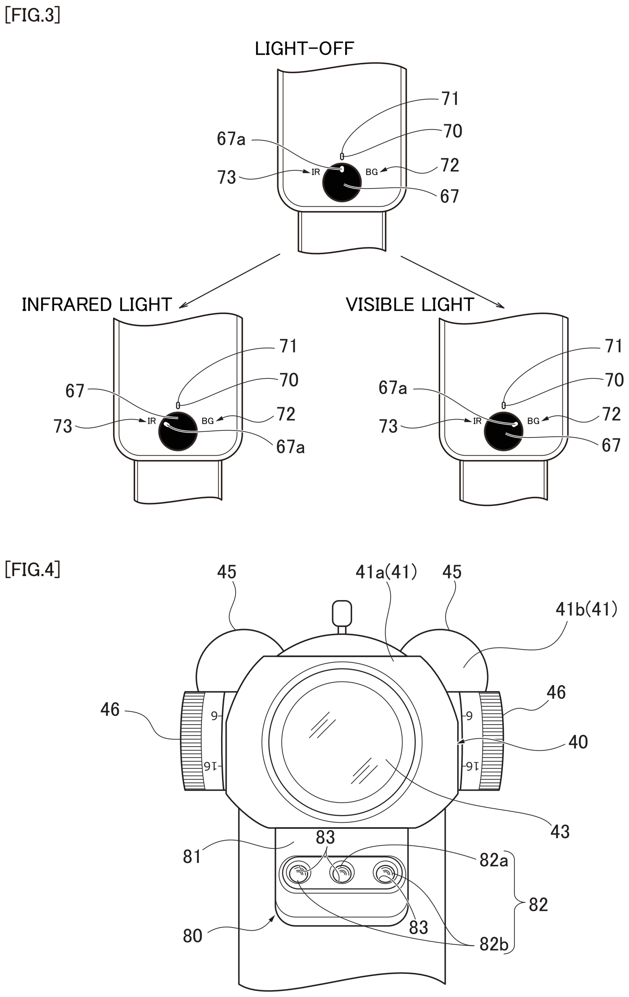

The LED light source of the LED board 66 serves as a light emitter 70, which is configured to indicate the status information to the examiner, including the power ON/OFF state of the imaging module 61 and the color of the background light emitted from the background illumination system 80. The light emitter 70 is off, which indicates the imaging module 61 is in a non-electrified state, i.e., in a stopped state (power off), and is lit in green, for example, which indicates the imaging module 61 is in an electrified state, i.e., in an activated state (power on) . The light emitter 70 is lit in blue, for example, which indicates that the background illumination system 80 emits visible light, and is lit in red, for example, which indicates it emits infrared light. It should be noted that the colors of the light emitted from the light emitter 70 are not limited to these example colors and may include other colors as long as the electrification state of the imaging module 61 and the color of the background light can be indicated. The light emitter 70 may also employ other emission modes or aspects, such as blinking. The illumination state and color of the light emitter 70 may be viewed through a display window 71 (see FIG. 3), provided on the examiner side of the imaging module housing 62, located in the middle above the background light switching unit 67.

The background light switching unit 67 allows the examiner to control the background illumination system 80 including turning the light on and off, switching the background light (between visible light and infrared light), and adjusting the brightness (amount of light) . The background light switching unit 67, as shown in FIGS. 1 to 3, is provided on one side of the imaging module housing 62, at a lower position of the examiner-facing side (back side) . The background light switching unit 67 has a marker 67a formed on its surface for indicating its rotational position. The display window 71 is formed on the imaging module housing 62, above the background light switching unit 67, at an upper position corresponding to the 12 o'clock. The position of the display window 71 serves as the reference position of the background light switching unit 67 and indicates the light-off position of the background illumination system 80. By rotating the background light switching unit 67 clockwise within a predetermined range, the background light source 82 emits visible light and increases its brightness to the maximum at the position (visible-light maximum position 72) indicated by the letters "BG"; conversely, by rotating counterclockwise within a predetermined range, the background light source 82 emits infrared light and increases its brightness to the maximum at the position (infrared-light maximum position 73) indicated by the letters "IR".

As shown in the top diagram of FIG. 3, where the marker 67a is at the light-off position, the LED board 66 controls the background light source 82 to turn off. As shown in the right diagram of FIG. 3, when the examiner rotates the background light switching unit 67 clockwise, the LED board 66 in turn controls the background light source 82 to emit visible light. At this time, the LED board 66 further controls the background light source 82 to increase the brightness of the visible light according to the operation amount (rotation amount) of the background light switching unit 67 from the light-off position to the position "BG", at which the brightness reaches its maximum.

Similarly, when the examiner rotates the background light switching unit 67 counterclockwise, the LED board 66 controls the background light source 82 to emit infrared light. At this time, the LED board 66 controls the background light source 82 to increase the brightness of the infrared light according to the operation amount (rotation amount) from the light-off position to the position "IR", at which the brightness reaches its maximum.

The background illumination system 80 illuminates, with background light, the region surrounding the area irradiated with slit light from the illumination system 30. The region illuminated by the background illumination system 80 may include at least the region surrounding the area irradiated with slit light, or may partially overlap with the area irradiated by the illumination system 30. The background illumination system 80 mainly comprises a background illumination housing 81, a background light source 82, and optical components such as a collecting lens (not shown) .

As shown in FIG. 4, the background light source 82 includes a visible light source 82a that emits visible light (background light: BG) and includes a pair of infrared light sources 82b that emits infrared light (IR) on both sides of the visible light source 82a. The visible light and the infrared light emitted from the background light source 82 can be turned on and off, switched alternately, and adjusted in brightness by operating the background light switching unit 67 disposed on the examiner side. The visible light may be employed for observation and for image capture of the eye itself, and the infrared light may be employed for observation and for image capture of a meibomian gland. The background light source 82 emits background light toward the eye E from an irradiation opening 83 formed on the background illumination housing 81. The background light emitted from the background light source 82 is converged by the collecting lens, and then emitted toward the eye E. It should be noted that the background light emitted from the background light source 82 may be both visible and infrared light, or either one of them. Although the background light source 8 light 2 includes the visible light source 82a and the infrared light source 82b in Embodiment 1, the background light source 82 may alternatively be configured as a single LED light source that emits either visible or infrared light alternately at a time.

The control device 50 controls the respective units and components of the slit-lamp microscope 100. The control device 50 is composed of a motherboard (main board) including a central processing unit (CPU) that serves as a control unit, a read-only memory (ROM), a random access memory (RAM), and a storage device, such as a hard disk drive. The control device 50 is disposed within the pedestal 12a.

The control device 50, which is connected to the power supply unit 20, the illumination system 30, the observation system 40, the imaging module 61, and the background illumination system 80 via the power cables w1 to w5, acquires necessary information from these components and provides corresponding control instructions to them as necessary. The control device 50 may include a storage device in which a control program is stored in advance. The control program is executed in cooperation with hardware resources, including a CPU, to process the acquired information and provide the control instructions. The control device 50 supplies power from the power supply unit 20 to the illumination system 30, the observation system 40, the imaging module 61, and the background illumination system 80.

The control device 50 may be connected to a device such as a PC, a tablet device, or a dedicated controller, each having an operation unit and a display unit. Upon receiving an operation from the examiner through the operation unit, the control device 50 controls the slit-lamp microscope 100 to output captured images and measurement results by displaying them on the display unit.

FIG. 2A illustrates the slit-lamp microscope 100 in a configuration without the imaging device 60. In this configuration, the second observation-system housing 41b, which houses part of the optical relay 44 and the eyepieces 45, is attached to the second supporting arm 14c. During installation, an installation technician attaches the second observation-system housing 41b to the second supporting arm 14c. This configuration, without the imaging device 60, allows the examiner to directly observe the eye E through the objective lens 43, the optical relay 44, and the eyepieces 45 of the observation system 40.

Alternatively, FIG. 2B illustrates the slit-lamp microscope 100 in a configuration with the imaging device 60. In this configuration, the imaging module 61 is attached to the second supporting arm 14c on the examiner side, and the background illumination system 80 is attached to the second supporting arm 14c at the attaching portion 14h on the subject-facing side. During installation, the installation technician connects the second power cable w2 extending from the imaging module 61 and the third power cable w3 extending from the background illumination system 80 each to the relay board 14g inside the second supporting arm 14c. The imaging module 61 and the background illumination system 80 are electrically connected to the control device 50, which allows the control device 50 to control the operations of these components.

The second observation-system housing 41b is then attached to the imaging module 61 on the examiner side. This configuration, with the imaging device 60, allows the examiner to directly observe the eye E as well as to capture an image of the eye E using the imaging module 61 together with the imaging module optical deflector 63, which deflects a portion of the return light from the eye E. The imaging unit 60 may be attached (or detached) by a manufacturer during production of the slit-lamp microscope 100, by a dealer or distributors upon delivery to a user, or by the user (the examiner).

As described above, the slit-lamp microscope 100 according to Embodiment 1 comprises the main unit 10 including the illumination system 30 that emits illumination light toward the eye E, and the observation system 40 for observing return light from the eye E; and the imaging unit 60 configured to be detachably attached to the main unit 10. The imaging unit 60 includes the imaging module 61 that captures an image of the eye E, and the specific light irradiation system (background illumination system 80) that emits specific light (background light), different from the illumination light, toward the eye E.

As described above, the imaging unit 60 is configured to be detachably attached to the main unit 10, and thus the slit-lamp microscope 100 according to Embodiment 1 can be used either with or without the imaging unit 60, as shown in FIGS. 2A and 2B, respectively, depending on the user needs or intended use. This detachable configuration of the imaging unit 60, including the background illumination system 80, also neither requires modification of nor affects other components, such as the observation system 40. Accordingly, the slit-lamp microscope 100 according to Embodiment 1 allows easy and selective attachment of the background illumination system 80 (specific light irradiation system) for emitting background light (specific light).

The main unit 10 according to Embodiment 1 further includes the second supporting arm 14c supporting the observation system 40, and the imaging unit 60 is configured to be detachably attached to the second supporting arm 14c. This configuration allows easier attachment and detachment of the imaging unit 60.

The observation system 40 according to Embodiment 1, which comprises the objective lens 43 facing the eye E and the ocular lens 45a facing the examiner's eye e, is configured to be attached to the upper part of the second supporting arm 14c. The background illumination system 80 (specific light irradiation system) is disposed on the subject-facing side of the second supporting arm 14c and below the objective lens 43. This configuration allows the background illumination system 80 to appropriately emit background light from its lower part toward the eye E. Thus, this background light facilitates the observation and image capture of the eye E in an more appropriate manner, and also reduces the likelihood that the subject will experience glare even when visible light is used as the background light.

Furthermore, in conventional slit-lamp microscopes, cables and wires are left exposed and visible, such as a power cable for the background illumination system and a communication cable for the imaging module, which extend to a power supply unit or to an external device outside from the slit-lamp microscope device. These cables may spoil the appearance and interfere with the movement of the main unit.

In contrast, the slit-lamp microscope 100 according to Embodiment 1 comprises a power supply unit 20 that supplies power, and the second supporting arm 14c has an accommodating space 14f that houses cables and wires such as the power cables w1 to w4, which connect the imaging unit 60 and the power supply unit 20. Also, the communication cable u1 passing through the accommodating space 14f connects to the imaging module 61 and the PC 2 serving as an external device to output an image captured by the imaging module 61 to the PC2. This configuration allows the cables of the slit-lamp microscope 100 according to Embodiment 1 to be housed in the accommodating space 14f of the second supporting arm 14c, thereby concealing them from the outside. Thus, the slit-lamp microscope 100 according to Embodiment 1 improves the accommodation of wiring, enhances the appearance, and ensures operability.

The second supporting arm 14c according to Embodiment 1 has the accommodating space 14f in which the relay board 14g is housed. The relay board 14g is connected to the first power cable (first wiring) w1 from the imaging module 61 and to the second power cable (second wiring) w2 from the background illumination system 80. The relay board 14g is further connected, to the third power cable w3 (third wiring) housed in the main unit 10, via the control device 50 and the fifth power cable w5, to the power supply unit 20. Accordingly, when the imaging unit 60 is to be attached to the main unit 10, the connection work simply involves connecting the second power cable w2 extending from the imaging module 61 and the third power cable w3 extending from the background illumination system 80 to the relay board 14g, thereby improving assembly efficiency. Furthermore, accommodating the third wiring entirely within the main unit 10 eliminates adverse effects on appearance and ensure operability.

Furthermore, conventional slit-lamp microscope ophthalmic devices typically have both the background illumination system and the operation unit disposed on the subject-facing side, which hinders the examiner from operating the operation unit. In contrast, Embodiment 1 has the imaging module 61 disposed on the second supporting arm 14c on the examiner-facing side, and the background light switching unit 67 (operation unit) of the background illumination system 80 disposed on the examiner-facing side of the imaging module 61. Accordingly, this configuration allows the examiner to easily operate the background light switching unit 67, thereby improving operability, including turning the background light on and off or switching it between visible and infrared light, and appropriately adjusting the brightness.

Furthermore, the imaging module 61 according to Embodiment 1 comprises an imaging module optical deflector 63 detachably arranged in the optical path of the observation system 40 to deflect a portion of return light out of the optical path of the observation system 40; and an imaging element 65 that receives the deflected return light deflected by the imaging module optical deflector 63. This configuration allows the imaging element 65 to capture images of the eye E substantially corresponding to that observed by the examiner. This configuration also allows the imaging module optical deflector 63 to be attached in and detached from the optical path of the observation system 40 in conjunction with the attachment and detachment of the imaging unit 60 to the main unit 10. Therefore, the observation optical system 42 of the observation system 40 needs no modification in configuration, and with the imaging module optical deflector 63 in place, the return light is directed toward the imaging element 65.

The background illumination system 80, serving as the specific light irradiation system according to Embodiment 1, emits background light as the specific light toward the eye E, in which the background light is at least either one of visible light and infrared light. This configuration allows the examiner to observe or capture images: using visible light, of the area surrounding the irradiated site; and using infrared light, of a meibomian gland, for example.

The imaging unit 60 of Embodiment 1 further includes, on the examiner-facing side, a light emitter 70 configured to emit light in a different aspect according to a color of background light emitted from the background illumination system 80. This configuration allows the examiner to easily recognize whether the background illumination system 80 is emitting visible light or infrared light, thereby facilitating more appropriate observation and image capture of the eye E.

### (Embodiment 2)

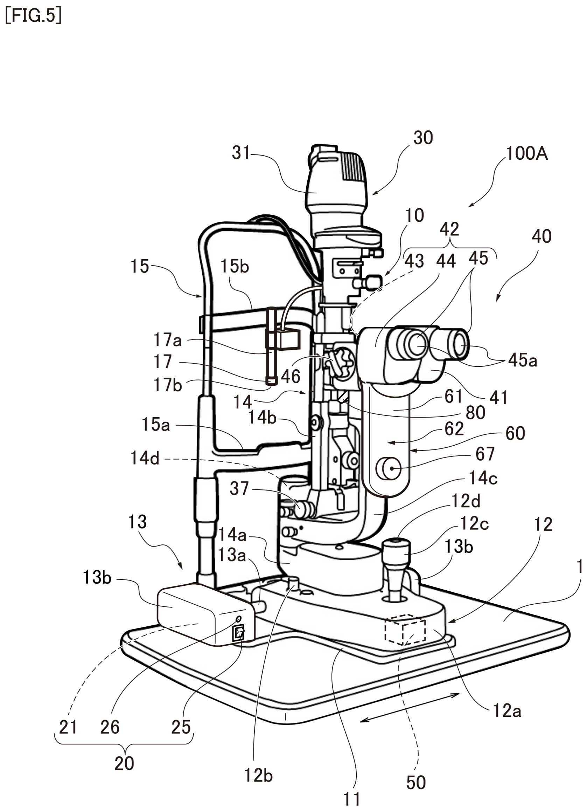

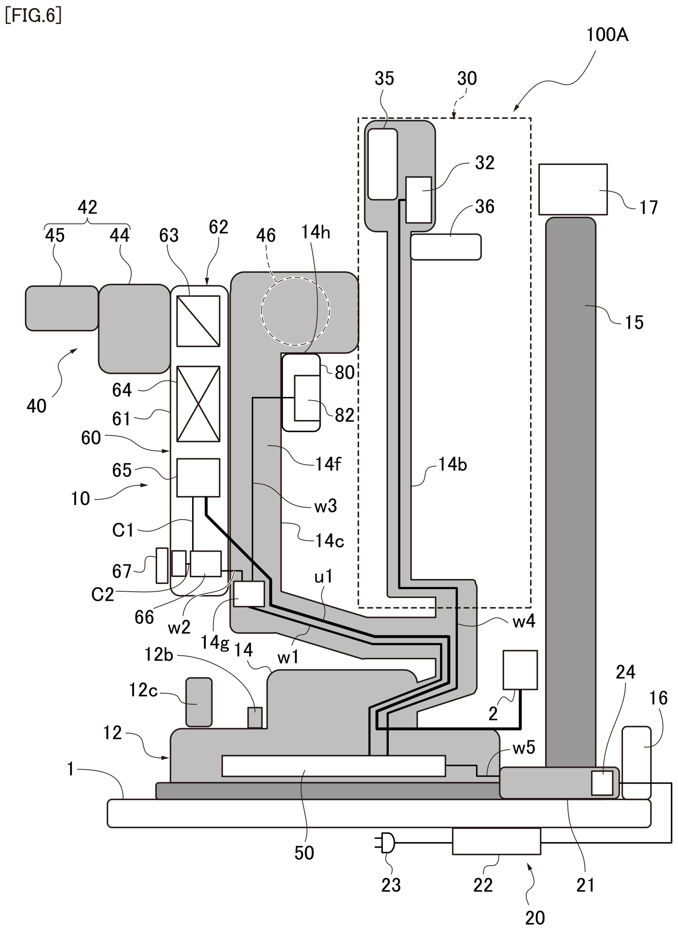

A slit-lamp microscope 100A according to Embodiment 2 is described below with reference to FIG. 5 and FIG. 6. The slit-lamp microscope 100A according to Embodiment 2 is a Haag-type (also referred to as a Goldmann-type) slit-lamp microscope, which has the same basic configuration as the slit-lamp microscope 100 according to Embodiment 1, except that the illumination system 30 is disposed above the observation system 40. As such, in the slit-lamp microscope 100A according to Embodiment 2, components having the same configuration as those in the slit-lamp microscope 100 according to Embodiment 1 are denoted by the same reference numerals as in Embodiment 1, and detailed explanations thereof are omitted.

As shown in FIG. 5 and FIG. 6, the slit-lamp microscope 100A according to Embodiment 2 mainly comprises a main unit 10 and a power supply unit 20. The main unit 10 mainly comprises an illumination system 30, an observation system 40, a control device 50, a base 11 mounted on the upper side of the optical table 1, a pedestal 12, a movement mechanism 13, a supporting part 14, and a face support 15.

The illumination system 30 is mounted on the upper part of a first supporting arm 14b and disposed above the observation system 40. The observation system 40 is mounted on the upper part of a second supporting arm 14c that has an accommodating space 14f therein.

The imaging unit 60 mainly comprises an imaging module 61 and a background illumination system 80 that emits visible light and infrared light selectively, and is detachably attached to the second supporting arm 14c of the main unit 10. FIG. 5 and FIG. 6 illustrate a configuration in which the imaging unit 60 is attached to the second supporting arm 14c. Also in Embodiment 2, the slit-lamp microscope 100A can be used without the imaging unit 60.

The imaging module 61 is attached to the examiner-facing side (back side) of the second supporting arm 14c. The background illumination system 80 is attached to the second supporting arm 14c at the attaching portion 14h on the subject-facing side (front side), and disposed below the objective lens 43 of the observation system 40. The imaging module housing 62 has the background light switching unit 67 and the light emitter 70 provided on the examiner side.

The imaging module 61 and the background illumination system 80 are connected to the relay board 14g via the first power cable w1 and the second power cable w2 each housed in the accommodating space 14f. The relay board 14g is connected to the control device 50 via the third power cable w3 housed in the accommodating space 14f and the pedestal 12. A communication cable u1 housed in the accommodating space 14f and the pedestal 12 connects the imaging element 65 and the PC 2.

The slit-lamp microscope 100A according to Embodiment 2 can also achieve the same effects as the slit-lamp microscope 100 according to Embodiment 1 described above. Similarly, in the slit-lamp microscope 100A according to Embodiment 2, the imaging unit 60 comprising the imaging module 61 and the background illumination system 80 as described in Embodiment 1 is provided detachably to the main unit 10. Accordingly, the slit-lamp microscope 100A according to Embodiment 2 allows easy and selective attachment of the background illumination system 80 (specific light irradiation system) for emitting background light (specific light).

The ophthalmic device of the present disclosure has been described above with reference to Embodiments 1 and 2. However, the specific configurations are not limited to these embodiments, and design modifications and additions may be made without departing from the scope and spirit of the invention as defined in the claims.

Although the ophthalmic device of these embodiments is directed to a slit-lamp microscope, the ophthalmic device to which the present disclosure is applied is not limited to a slit-lamp microscope. The ophthalmic device may be a surgical microscope. Apart from these devices, the present disclosure can be applied to any ophthalmic device that performs observation and image capture of an eye under examination using specific light different from illumination light. In this instance, the specific light irradiation system is not limited to a configuration that emits background light, and may also have a configuration that emits second illumination light, whose wavelength, illumination range, and the like are different from the illumination light for observation.

Although the light emitter 70 according to each of the above embodiments is implemented as an LED light source of the LED board 66 in the imaging module housing 62, emits light through the display window 71, and change the light according to the conditions of the background light, the light emitter 70 is not limited to this configuration. For example, the light emitter 70 may be also provided on the back surface of the letters "BG" and "IR" indicating a visible light maximum position 72 and an infrared light maximum position 73 respectively, so as to emit light from these letters in a predetermined color or aspect according to an operation of the background light switching unit 67. Alternatively, the light emitter 70 may be also provided on the marker 67a part in the background light switching unit 67 emit light from the marker 67a in a predetermined color or aspect according to the operation of the background light switching unit 67.

### [Cross-reference to related applications]

This application claims priority based on Japanese Patent Application JP2023-092438 filed with the Japan Patent Office on June 5, 2023. The entire disclosure of this application is hereby incorporated by reference herein in its entirety.

## Claims

1. An ophthalmic device comprising:
a main unit including:
an illumination system configured to emit illumination light toward an eye of a subject under examination, and
an observation system configured to observe return light from the eye; and
an imaging unit configured to be detachably attached to the main unit,
wherein the imaging unit comprises:
an imaging module configured to acquire an image of the eye; and
a specific light irradiation system configured to emit specific light, different from the illumination light, toward the eye.

2. The ophthalmic device according to claim 1,
wherein the main unit further includes a supporting arm that supports the observation system, and
wherein the imaging unit is configured to be detachably attached to the supporting arm.

3. The ophthalmic device according to claim 2,
wherein the observation system comprises an objective lens facing the eye and an ocular lens facing an examiner's eye, and is configured to be attached to the upper part of the supporting arm, and
wherein the specific light irradiation system is disposed on the subject-facing side of the supporting arm as well as below the objective lens.

4. The ophthalmic device according to claim 2,
wherein the ophthalmic device comprises a power supply unit that supplies power,
wherein the supporting arm has an accommodating space that houses wiring, and
wherein the imaging unit is connected to the power supply unit via the wiring housed in the accommodating space.

5. The ophthalmic device according to claim 4,
wherein the supporting arm has a relay board disposed in the accommodating space, the relay board being connected a first wiring from the imaging module, a second wiring from the specific light irradiation system, and to the power supply unit through a third wiring housed in the main unit.

6. The ophthalmic device according to claim 2,
wherein the imaging module is disposed on the examiner-facing side of the supporting arm, and
wherein an operation unit of the specific light irradiation system is provided on the examiner-facing side of the imaging module.

7. The ophthalmic device according to claim 1,
wherein the imaging module comprises:
an optical deflector configured to be arranged in an optical path of the observation system and to deflect a portion of return light out of the optical path of the observation system; and
an imaging element that receives the return light deflected by the optical deflector.

8. The ophthalmic device according to claim 1,
wherein the specific light irradiation system further configured to emit at least one of visible light and infrared light as the specific light toward the eye.

9. The ophthalmic device according to claim 8,
wherein the imaging unit further includes a light emitter configured to emit light in a different aspect according to a color of the specific light emitted from the specific light irradiation system, the light emitter being disposed on the examiner-facing side.
